**Europäisches Patentamt**

**European Patent Office**

**Office européen des brevets**

(11) Publication number: **0 657 543 A1**

# EUROPEAN PATENT APPLICATION
## published in accordance with Art. 158(3) EPC

(21) Application number: **93906857.3**

(51) Int. Cl.⁶: **C12P 7/64**

(22) Date of filing: **31.03.93**

(86) International application number:
**PCT/JP93/00402**

(87) International publication number:
**WO 93/20225 (14.10.93 93/25)**

(30) Priority: **31.03.92 JP 77189/92**
**05.03.93 JP 44764/93**

(43) Date of publication of application:
**14.06.95 Bulletin 95/24**

(84) Designated Contracting States:
**DE FR GB**

(71) Applicant: **KAWASAKI STEEL CORPORATION**
**No. 1-28, 1-Chome Kitahonmachi-Dori**
**Chuo-Ku, Kobe-City Hyogo 651 (JP)**

(72) Inventor: **TAKEUCHI, Daizo, Kawasaki Steel**
**Corporation**
**Techn. Res. Div,**
**1-Banchi,**
**Kawasaki-cho,**
**Chuo-ku**
**Chiba-shi,**
**Chiba 260 (JP)**
Inventor: **Lizuka, Tokio, Kawasaki Steel**
**Corporation**
**Techn. Res. Div.,**
**1-Banchi,**
**Kawasaki-cho,**
**Chuo-ku**
**Chiba-shi,**
**Chiba 260 (JP)**
Inventor: **UEHARA, Kenichi, Kawasaki Steel**
**Corporation**
**Techn. Res. Div.,**
**1-Banchi,**
**Kawasaki-cho,**
**Chuo-ku**
**Chiba-shi,**
**Chiba 260 (JP)**

(74) Representative: **Sanderson, Nigel Paul**
**Haseltine Lake & Co.**
**Hazlitt House**
**28, Southampton Buildings**
**Chancery Lane**
**London WC2A 1AT (GB)**

(54) **PROCESS FOR PRODUCING DOCOSAHEXAENOIC ACID.**

(57) A process for producing docosahexaenoic acid by subjecting *Crypthecodinium cohnii*, one of marine microalgae, to liquid shake culture, liquid submerged aerobic spinner culture and/or aerobic spinner culture in the presence of a surfactant and/or a defoamer; and a process for producing docosahexaenoic acid by subjecting *Crypthecodinium cohnii* ATCC30021 strain to liquid shake culture, liquid submerged aerobic spinner culture and/or aerobic spinner culture.

TECHNICAL FIELD

This invention relates to a process for the production of a highly unsaturated fatty acid, docosahexaenoic acid, which is possessed of noteworthy physiological functions such as a cholesterol level reducing effect, an anticoagulant effect and a carcinostatic effect, as well as its brain metabolism-related application to the improvement of memory and learning capacities, prevention of senile dementia and treatment of Alzheimer disease and its use as an essential fatty acid for the growth of juvenile fish.

BACKGROUND ART

It is known that docosahexaenoic acid shows various physiologically active functions as one of fish oil components, and, triggered by the finding of a material which contains docosahexaenoic acid in high concentration (tuna orbit fat) and the development of new highly efficient purification techniques in recent years, studies are being conducted in earnest at many research institutions on the elucidation of the physiologically active functions of docosahexaenoic acid. With regard to the purification and use of docosahexaenoic acid, a large number of reports have been published, such as on an agent for the prevention and treatment of thrombosis (Japanese Patent Application Kokai No. 57-183716), a process for the separation and purification of highly unsaturated fatty acids or esters thereof (Japanese Patent Application Kokai No. 61-37752), a carcinostatic composition containing a highly unsaturated fatty acid (Japanese Patent Application Kokai No. 63-258816), a process for the separation and purification of highly unsaturated fatty acid alkyl esters (Japanese Patent Application Kokai No. 63-8356), a carcinostatic agent containing docosahexaenoyl monoglyceride as an active ingredient (Japanese Patent Application Kokai No. 1-203322), a brain function enhancing agent containing docosahexaenoic acid and its esters as active ingredients (Japanese Patent Application Kokai No. 1-153629) and a brain function improving composition, a learning capacity increasing agent, a memorial power increasing agent, a dementia preventing agent, a dementia treating agent or a physiologically functional food having brain function improving effects (Japanese Patent Application Kokai No. 2-49723).

With regard to the production process of docosahexaenoic acid, on the other hand, its materialization has been delayed because of a difficulty in separating docosahexaenoic acid from other highly unsaturated fatty acids having similar physical properties, such as arachidonic acid, eicosapentaenoic acid and the like, when docosahexaenoic acid is extracted as a fatty acid from tuna, sardines, bonito and the like fish oils.

As an alternative method other than its extraction and purification from fish oil, its selective production by microorganisms and the like has been examined, seeking for other sources than fish oil. Especially, studies have been conducted for a long time on the production of docosahexaenoic acid by propagating *Crypthecodinium cohnii* which belongs to a marine microalga.

Media for use in the propagation of marine microalgae such as *Crypthecodinium cohnii* are roughly divided into two groups, namely a natural medium in which sea water used as a base is further supplemented with deficient nutrients and a synthetic medium whose components are defined.

However, the former medium have substantially unavoidable disadvantages in that precipitate is formed frequently by its high pressure sterilization and its physiological properties change depending not only on the sea water used but also on the season and preservation period even if the sea water is collected at the same area. In order to resolve such problems, a concentrated artificial sea water is used broadly, but with other drawbacks in that it contains slightly soluble components and it can hardly be applied to the examination of medium composition due to its unstable contents.

On the other hand, the latter medium does not form precipitate by high pressure sterilization and its actual reproducibility is guaranteed, but it is necessary to select a specific medium suitable for the physiological requirement of each marine microalga (M. Chihara and K. Nishizawa: *Methods for Studying Algae*, Kyohritsu Shuppan, 281 - 305, 1979).

Among several reports on the culturing of *Crypthecodinium cohnii* , R.J. Henderson *et al*. have examined and reported on the biosynthesis of $^{14}$C-labeled docosahexaenoic acid in *Crypthecodinium cohnii* using AXM medium as a synthetic medium (*Phytochemistry*, 27 (6), 1679 - 1683 (1988)), but showing only about the occupying ratio of docosahexaenoic acid in triglyceride and phospholipid fractions with no description on the actual yield of docosahexaenoic acid.

Also, R.C. Tuttle, R.C. *et al*. have carried out optimization of medium components using a synthetic MLH medium as a basal medium with the aim of optimizing growth rate of algal cells and reported on the effect of the optimization on the generation time (*Phycologia*, 14 (1), 1 - 8 (1975)), but with no description about influence and effect of each medium component on the composition and contents of fatty acids such as docosahexaenoic acid and the like.

In addition, MARTEK CORPORATION has examined and reported on the culturing of algae using a medium prepared by supplementing a natural or artificial sea water used as the base with glucose and yeast extract, which has been carried out with the aim of improving docosahexaenoic acid yield (WO 91/11918). However, it does not discloses effect of each component of the synthetic medium on the growth of algal cells and accumulation of docosahexaenoic acid.

On the other hand, the inventors of the present invention have reported in Japanese Patent Application No. 04-77189 and Japanese Patent Application No. 04-344279 that stable growth of an alga and high productivity of docosahexaenoic acid were obtained when a marine microalga was inoculated into a medium containing specified sugars, organic nitrogen sources, inorganic salts and heavy metal elements as essential components.

In addition to the above, several processes have been disclosed such as a process in which a highly unsaturated fatty acid is produced by microbial conversion using a strain belonging to the genus *Mortierella* (Japanese Patent Application Kokai No. 63-185389), a process in which a highly unsaturated fatty acid-enriched oil is produced by a microorganism capable of producing arachidonic acid (Japanese Patent Application Kokai No. 1-304892), a process in which a highly unsaturated fatty acid-containing lipid is produced from a marine microorganism (Japanese Patent Application Kokai No. 2-142486), a process in which a highly unsaturated fatty acid is produced by a strain belonging to the genus *Echinosporangium* - (Japanese Patent Application Kokai No. 2-23878) and a process in which a highly unsaturated fatty acid is obtained from culture mixtures of *Echinosporangium transversalie* ATCC 16960 and 18036 (European Patent Application 355972).

According to the examination carried out by Pratima Bajpai *et al.*, production of docosahexaenoic acid by a lower fungus *Thraustochytrium aureum* is reported (*Appl. Microbiol. Biotechnol.*, <u>35</u>, 706 (1991)), but this process has disadvantages in that it requires a specific and large-scale culturing apparatus and a high-grade separation purification apparatus, because light is required for the culturing of this fungus and other highly unsaturated fatty acids having similar physical properties, such as arachidonic acid and eicosapentaenoic acid, are simultaneously produced in a yield of 10 to 20%.

In addition, according to the examination carried out by R.J. Henderson *et al.*, a marine microalga *Crypthecodinium cohnii* produces docosahexaenoic acid almost solely as a highly unsaturated fatty acid in an amount of about 9% based on the total fatty acids (*Phytochemistry*, <u>27</u> (6), 1697 (1988)), but with drawbacks in that the standing culture method used for the culturing is not suitable for large scale culturing and the docosahexaenoic acid content is small.

In this connection, only a process for the production of docosahexaenoic acid by *Crypthecodinium cohnii* MK-8840 (ATCC 40750) and a synthetic product thereof (WO 91/11918), effects of environmental conditions on the formation of docosahexaenoic acid in *Crypthecodinium cohnii* (D.H. Beach *et al.*; *Biochimica et Biophysica Acta*, <u>316</u>, 56 - 65 (1975)) and a book: David J. Kyle *et al.*; *Industrial Application of Single Cell Oils*, (American Oil Chemist's Society, 1992) chapter <u>16</u>; 287 - 300 are known to date, but the productivity by these disclosed techniques is too low for stable industrial production. Since these known facts are clearly different from the process of the present invention which renders possible stable production of docosahexaenoic acid with a high concentration, and there are many technical problems to be resolved for the purpose of materializing these already known processes, it is difficult to apply these prior art techniques to industrially practical production processes from the viewpoint of economic profitability.

There are other known processes in which microorganisms are used as docosahexaenoic acid producers, such as a bacterium (Delong, E.F. & Yayanos, A.A.: *Appl. Environ. Microbiol.*, <u>51 (4)</u>, 730 (1986)), a fungus *Thraustochytrium* (Haskins, R.H. *et al.*; *Can. J. Microbiol.*, <u>10</u>, 187 (1964)), a fungus *Entomophthora* (Tyrrell; *Can. J. Microbiol.*, <u>13</u>, 755 (1967)) and a fungus *Japonochytrium* sp. ATCC 28207 (Japanese Patent Application Kokai No. 1-199588), as well as processes in which algae such as Dinoflagellates, Haptophyceae and the like are used (Joseph, J.D.; *Lipids*, <u>10</u>, 395 (1975), Nichols, P.D. *et al.*, *Phytochemistry*, <u>23</u>, 1043 (1984)), but these processes are based on natural propagation or simple standing culture with no positive attempts to increase docosahexaenoic acid content in algal cells and therefore have many technical problems remained unsolved for their materialization.

DISCLOSURE OF THE INVENTION

Though docosahexaenoic acid as the compound of interest of the present invention is expected to have various physiological activities such as carcinostatic effects, its supply and quality are not stable because its material is dependent upon fish oil. In addition, in spite of the use of many purification steps and cost in order to remove the fish oil-specific odor, the fishy odor cannot be removed sufficiently hence entailing inconvenient applicability of the product, and, because of these technical problems still remained unsolved, the final product becomes disadvantageously expensive. In order to resale these problems, the present invention provides a process for the stable and low cost production of docosahexaenoic acid in which a material source other than fishes is used.

In addition to the above, in relation to the production of docosahexaenoic acid by its extraction from algal cells of a marine microalga, great concern has been directed toward the development of simple and effective seed culture quantity controlling method and medium composition and an optimum culture condition so that the content of docosahexaenoic acid in lipids can be improved further and it can be produced stably.

BEST MODE OF CARRYING OUT THE INVENTION

With the aim of overcoming these problems, the inventors of the present invention have made intensive attempts and found a process in which a marine microalga selected as a strain capable of producing and supplying docosahexaenoic acid industrially stably with a constant quality is subjected to liquid shaking culture, liquid submerged aeration agitation culture and/or aeration agitation culture in the presence or absence of a surfactant and/or an antifoaming agent, thereby effecting sharp increment of the content of docosahexaenoic acid in algal cells, increment of the productivity of algal cell and stable and low cast production of docosahexaenoic acid through its extraction from the algal cells thus obtained.

In addition, a success was attained in producing algal cells at a high concentration by subculturing marine microalgae whose cells are propagated by standing culture in the prior art, in the presence of a surfactant and/or an antifoaming agent in order to enrich an antifoaming agent resistant strain or a surfactant resistant strain, and subjecting the strain to liquid shaking culture or liquid submerged aeration agitation culture and/or aeration agitation culture in the presence of a surfactant and/or an antifoaming agent. Also, the present inventors have succeeded in selecting and breeding an algal strain having sharply increased productivity, through the repetition of single cell isolation from the antifoaming agent resistant or surfactant resistant strain and acclimation culture of the isolated cells, by controlling inoculum size of the seed culture at a constant level and restricting the agitation blade peripheral speed to a lower level of a specified rate.

It has been considered that *Crypthecodinium cohnii* is difficult to be cultured except for standing culture, because agitation by agitation blades causes death of marine microalgae due to shearing of the cells. On the other hand, it has been shown recently by MARTEK CORPORATION that DHA could be produced even by the use of agitation culturing. However, it cannot be said yet that the productivity is sufficient. In addition, it was difficult to obtain DHA stably in a high concentration because of the sensitivity to various surfactants and/or antifoaming agents used in microbial fermentation. However, the present invention has been accomplished based on the success in discovering and breeding a *Crypthecodinium cohnii* strain which is resistant to these agents, as the result of the repetition of single cell isolation of resistant strains having growing capacity and acclimation culture thereof.

It provides a medium composition and a culture condition by which the content of docosahexaenoic acid in lipids of such a marine microalga can be increased to about 40%. The present invention has been accomplished by further finding that stable growth of algal cells and increased content of docosahexaenoic acid in lipids can be attained when the marine microalga is cultured in a medium in which a surfactant and/or an antifoaming agent is allowed to exist.

Thereafter, a process for obtaining docosahexaenoic acid was completed in which the algal cells are recovered and docosahexaenoic acid is extracted and purified from the wet algal cells as such or after drying.

The algae to be used in the present invention are marine microalgae, especially an alga belonging to *Crypthecodinium cohnii* or the like. These are known algae and can be obtained from stock culture organizations including ATCC (American Type Culture Collection), and their illustrative examples include *Crypthecodinium cohnii* ATCC 30021, 30543, 30556, 30571, 30572, 30575, 50051, 50053, 50055, 50056, 50058 and 50060. Of these, *Crypthecodinium cohnii* ATCC 30021 is particularly preferred because of its high DHA productivity.

Also, it is preferable to use an algal strain which is obtained by bleeding into a surfactant resistant and/or antifoaming agent resistant strain through the repetition of single cell isolation and acclimation culturing.

In order to breed such a strain, cultured algal cells are diluted with 2% physiological saline by a factor of $10^2$ to $10^3$, spread on an agar medium containing 2% by weight of glucose, 0.2% by weight of yeast extract and 0.01 to 1.0 g/L of a surfactant and/or an antifoaming agent, followed by 3 to 4 days of incubation at 28°C to form colonies. A colony thus formed is picked up and cultured on a shaker using a liquid medium which contains 0.01 to 1.0 g/L of a surfactant and/or an antifoaming agent to collect the thus grown algal strain, the same procedure is repeated 10 to 15 times to select an algal strain which is resistant against the surfactant and/or antifoaming agent and has a high docosahexaenoic acid productivity, and cells of the thus obtained algal strain are cultured in a jar fermenter and the resulting culture broth is subjected to the same procedure which is repeated 5 to 10 times, thereby effecting breeding and isolation of an antifoaming agent resistant and/or surfactant resistant strain.

In addition to this, the use of mutant strains of said microorganism obtained by known mutagenesis for example by its ultraviolet ray irradiation or treatment with various mutagens is also included in the present invention.

Any medium composition may be used for the culturing of the alga to be used in the present invention, provided that the alga can propagate themselves properly in the medium. Appropriate carbon sources, nitrogen sources, inorganic salts and the like may be contained as the medium components. As such carbon sources, optional carbon sources which can be assimilated by the alga of the present invention may be used. Illustrative examples of organic materials which can be used as such carbon sources include organic compounds such as glycerol and the like, carbohydrates such as glucose, fructose, galactose and the like and organic acids such as acetic acid and the like. When such carbon sources are used, the docosahexaenoic acid content can be increased markedly without containing highly unsaturated fatty acids other than docosahexaenoic acid.

Illustrative examples of the nitrogen sources include usually used inorganic nitrogen compounds such as ammonium sulfate, ammonium nitrate and the like and organic nitrogen sources such as peptone, yeast extract, casein hydrolysate, glutamic acid, corn steep liquor and the like.

With regard to the inorganic salts, natural sea water may be the best, but various types of artificially prepared known artificial sea water may also be used, as well as various sodium salts, phosphates, magnesium salts, potassium salts, borates, carbonates and the like. Also useful are trace amounts of heavy metal salts such as iron salts, manganese salts, cobalt salts, zinc salts, chloride compounds, bromine compounds and the like.

As for the culturing method, the present inventors have made intensive attempts to carry out the culturing more efficiently which has been carried out only by standing culture in the prior art and found that the culturing efficiency can be improved sharply by employing liquid shaking culture, liquid submerged aeration agitation culture and/or aeration agitation culture in comparison with the conventional standing culture, namely about 1.5 times improvement in the yield of algal cells after 7 days of culturing and 1.3 to 6 times improvement in the ratio of docosahexaenoic acid content in the algal cells, and further 2 to 25 times improvement in the algal cell yield with 1.3 to 6 times improvement in the ratio of docosahexaenoic acid content in the algal cells in the presence of a surfactant and/or an antifoaming agent.

Liquid shaking culture is also called suspension culture in which an alga capable of producing docosahexaenoic acid is inoculated into a liquid medium and cultured on a shaker with continuous shaking. This method may be effected by the use of an apparatus which shakes culture flasks reciprocally or rotates them horizontally or an apparatus which keeps long and slender culture tubes horizontally and reciprocates both ends of each tube upward and downward. Rotary tube culture is also included in the liquid shaking culture.

Liquid submerged aeration agitation culture is also called tank culture in which a liquid medium is put in a closed tank preferably made of stainless steel and sterilized and then an alga of interest to be cultured is inoculated and cultured by revolving an agitator and aerating sterile air. The aeration rate is set generally to 0.2 to 1.5 l/l/min. In the case of aeration agitation culture, it may be effected by air agitation culture using an air lift type fermenter. The usual atmosphere may be used as the air which may be further enriched with oxygen. The concentration of dissolved oxygen may be in the range of approximately from 2 to 50%, because the amount if smaller than 2% would cause slow growth and if larger than 50% would entail reduction of the algal cell yield.

In addition, in the case of the liquid submerged aeration agitation culture, it is preferable to set the agitation blade peripheral speed to 250 cm/sec or less. An agitation blade peripheral speed exceeding 250 cm/sec is not practical because the number of dead algal cells caused by the shearing of marine microalgal cells increases and their propagation ability decreases considerably.

Culturing may be carried out at a temperature of from 15 to 34°C, optimally at 25 to 32°C. The medium pH may be within a neutral range, and the culturing period may be approximately 2 to 12 days in general, though it should be decided based on the amount of remaining carbon sources and the amount of substances secreted into the medium.

In the practice of the culturing of the present invention, in case of a surfactant such as a fatty acid ester base agent or an antifoaming agent such as a silicone based surfactant or a mixture thereof is added to the medium, further preferable productivity is obtained. Examples of the surfactant include glycerine monofatty acid esters such as glycerine monooleate, glycerine monostearate, glycerine monopalmitate, glycerine monolinolate and the like and sorbitan fatty acid esters such as sorbitan monostearate and the like. Illustrative examples of the antifoaming agent include Silicon KM-75 and the like. Since surfactants are also useful as antifoaming agents, surfactants may be used alone or as a mixture of two or more.

The surfactant to be used in the present invention may have an HLB (hydrophile lipophile balance) value of 16 or less, preferably from 0 to 15, more preferably from 3 to 5, based on the formula of Davis. The effect of the addition of the surfactant is small when the HLB value exceeds 16, because the docosahexaenoic acid productivity becomes close to the case of no addition. Though they are not surfactants, triglycerides having negative values of HLB show certain effects but with small effect of their addition.

Illustrative examples of the surfactant include nonionic surfactants whose HLB values are within the aforementioned range, such as Tween 80 and Tween 85 manufactured by Atlas Powder Co., Span 80 and Span 85 manufactured by Atlas Powder Co., oleyl alcohol, oleic acid monoglyceride, oleic acid triglyceride and the like.

The surfactant and/or antifoaming agent may be used in the medium in an amount of from 0.01 to 1.0 g/L, preferably from 0.05 to 1.0 g/L. Effect of the addition of the surfactant and/or antifoaming agent is extremely small when the concentration is smaller than 0.01 g/L. Also, the concentration larger than 1.0 g/L is not preferable because, though it will bear the adding effect of the surfactant and/or antifoaming agent, it spoils efficiencies of the recovery of algal cells and purification of docosahexaenoic acid.

In the prior art, it was not sure whether or not the addition of a surfactant and/or an antifoaming agent as employed in the inventive culturing method can be applied to the culturing of marine microalgae to be used in the present invention.

On the other hand, in accordance with the culturing method of the present invention, addition of a surfactant and/or an antifoaming agent is effective not only in preventing foaming but also in increasing the yield of algal cells by a factor of 20 to 30%, with the best case of 50%, in comparison with the prior art method, and renders possible stable culturing. Still more, with the increase in the algal cells, the yield of DHA also increases by a factor of 20 to 30%, with the best case of 50%, in comparison with the prior art method.

Preferably, the amount of a culture broth (inoculum size of seed culture) to be added to the initial medium of the culturing may be in the range of from 1.5 to 20% by weight based on the weight of the initial medium (about 1.5 to 20 ml per 100 ml of the initial medium). The amount if smaller than 1.5% by weight would bear no sufficient amount of algal cells. Also, the amount if larger than 20% by weight would result in poor culturing efficiency due to almost constant final algal cell concentration per unit time.

After completion of the culturing, algal cells are recovered from the culture broth by usually used centrifugation method preferably at a temperature equal to or lower than the culture temperature, for example by 10 to 15 minutes of centrifugation at 5 to 15°C and at 5,000 to 12,000 x g. The thus recovered algal cells are used as intact and wet algal cells with no further treatment or after making them into dry algal cells by freeze drying or heat aeration drying.

Docosahexaenoic acid can be extracted from the algal cells. Alternatively, crude lipids containing docosahexaenoic acid in a high concentration may be used by extracting the lipids from the wet or dry algal cells. Extraction and purification of docosahexaenoic acid from the algal cells may be carried out in the presence of a fatty acid oxidation inhibitor such as $\alpha$-tocopherol, or by a process in which lipids are extracted in an atmosphere of nitrogen using an organic solvent system such as methanol/chloroform or the like or in accordance with the purification method of Folch, Lees or Stanley. The thus partially purified product may be further purified for example by various types of column chromatography or recrystallization.

Since the crude lipids obtained from the algal cells hardly contain highly unsaturated fatty acids, such as arachidonic acid and eicosapentaenoic acid, whose physical properties are very close to those of docosahexaenoic acid, docosahexaenoic acid can be obtained markedly simply and efficiently in comparison with the prior art purification from fish oil and the like.

Since docosahexaenoic acid produced by the process of the present invention is free from the fish-specific smell and can be purified easily, it is useful as healthy food and physiologically active substance.

EXAMPLES

The following examples are provided to further illustrate the present invention.

(Inventive Example 1)

A loopful of the cells of *Crypthecodinium cohnii* ATCC 30021 were inoculated into 100 ml of medium (I) shown in Table 3 to carry out 7 days of standing culture at 28°C. A 10 ml portion of the thus obtained culture medium was inoculated as a seed culture into each of 10 flasks of 300 ml capacity containing 100 ml of medium (I) and cultured at a temperature of 28°C, pH 6.8, for 5 days on a rotary shaker at 180 r.p.m., and, after completion of the culturing the resulting culture broths in the 10 flasks were combined and subjected to 15 minutes of centrifugation at 12,000 x g and at 5°C to separate algal cells. Thereafter, the algal cells were washed with water and centrifuged in the same manner, and the thus separated algal cells were dried at 105°C for 5 hours to obtain 1.8 g per 1 L medium of dry algal cells.

Extraction and purification of docosahexaenoic acid from 1.8 g of the dry algal cells were carried out in accordance with the commonly used Bligh-Dyer extraction method (*Shin Seikagaku Jikken Koza*, 4, Lipid II, p.9 (1991) Tokyo Kagaku Dojin) using a Waring blender in which the algal cells were mixed with 20 volumes of methanol/chloroform (1/2), thereby obtaining a partially purified product (DHA content was 34.5% based on the total lipids). This was subjected to methylesterification (the just described publication, p.54) in order to identify the product by a gas chromatographic analysis using an authentic sample of docosahexaenoic acid as a standard reference material. Separation was carried out by a silica gel thin layer chromatography in the usual way (the same publication, p.12 and p.30) to obtain 242 mg of docosahexaenoic acid with a purity of 99.2% by weight. Separation was also carried out by a liquid chromatography (column, ODS; mobile phase, methanol: water = 95:5; flow rate, 1.0 ml/min; detection, RI) to obtain 250 mg of docosahexaenoic acid with a purity of 99.7% by weight.

Fatty acid composition in the algal cells determined by a gas chromatography was as follows.

| Fatty acid | Standing culture algal cells | Shake culture algal cells |
|---|---|---|
| C 12:0 | 33.4% | 5.7% |
| C 14:0 | 40.2% | 27.5% |
| C 16:0 | 10.9% | 22.7% |
| C 18:0 | 0.9% | 1.6% |
| C 18:1 | 3.3% | 8.0% |
| C 22:6 | 11.3% | 34.5% (docosahexaenoic acid) |
| | 100.0% | 100.0% |

(Inventive Example 2)

Culturing was carried out under the same conditions as described in Inventive Example 1, except for the use of a medium prepared by adding 5 mg of sorbitan fatty acid ester to the medium (I) at the time of shaking culture. Yield of the thus obtained algal cells was 2.1 g as dry algal cells, amount of total lipids obtained from the algal cells was 0.65 g and amount of docosahexaenoic acid was 292 mg.

Fatty acid composition in the algal cells determined by a gas chromatography was as follows.

| Fatty acid | Standing culture algal cells | Shake culture algal cells (addition of antifoaming agent) |
|---|---|---|
| C 12:0 | 33.4% | 5.2% |
| C 14:0 | 40.2% | 21.2% |
| C 16:0 | 10.9% | 20.4% |
| C 18:0 | 0.9% | 1.4% |
| C 18:1 | 3.3% | 6.0% |
| C 22:6 | 11.3% | 45.5% (docosahexaenoic acid) |
| | 100.0% | 100.0% |

(Inventive Example 3)

A 10 ml portion of cultured broth of *Crypthecodinium cohnii* ATCC 30021 obtained by a standing culture was inoculated into each of 5 flasks of 300 ml capacity containing 100 ml of medium (I) having the same composition as used in Inventive Example 1 and cultured at 28°C on a rotary shaker at 180 r.p.m. The resulting culture broths in 5 flasks were combined and 500 ml of the mixture was inoculated as a seed into a 10 L capacity jar fermenter containing 7 L of the medium (I) to carry out 5 days of culturing. The culturing was carried out at a temperature of 28°C, at a pH level of 7.0, at an agitation blade peripheral speed of 120 cm/sec and at an aeration rate of 3.5 l/min (medium volume/aeration volume/minute, 0.5 V.V.M.). As an antifoaming agent, 0.75 g of sorbitan fatty acid ester was used. After completion of the culturing, algal cells were recovered by 15 minutes of centrifugation at 12,000 x g. The cell yield was 43.4 g after 5 hours of drying at 105°C. The thus obtained dry cells were extracted and treated in the same manner as described in Inventive Example 1 to obtain 5.5 g of docosahexaenoic acid (DHA content in total lipids, 45.5%).

(Inventive Example 4)

Culturing was carried out in the same manner as described in Inventive Example 3, except that, in the 10 L capacity jar fermenter culturing, glucose as a carbon source, yeast extract as a nitrogen source and minerals were aseptically supplied 60, 80 and 100 hours after commencement of the culturing in such a way that the carbon source was used in a total of 10%, by supplying the medium (I) of Table 3 in which the amount of glucose was changed to 200 g and casein hydrolysate was replaced by the same amount of yeast extract. The culture temperature was kept constant at 28°C, but the agitation speed was increased from 120 cm/sec to 140 cm/sec stepwisely at each time of the aforementioned medium supply and the aeration rate was also increased from 0.3 V.V.M. to 1.0 V.V.M. in the same manner, using 2.25 g of the antifoaming agent. After 120 hours of the culturing, 184.1 g of dry algal cells were obtained. The thus obtained dry algal cells were extracted and treated in the same manner as described in Inventive Example 1 to obtain 23.8 g of docosahexaenoic acid (DHA content in total lipids, 40.0%).

(Inventive Example 5)

The culturing of Inventive Example 4 was repeated, except that 2.0 g of oleic acid monoglyceride was added as an antifoaming agent (surfactant), to obtain 176.7 g of dry algal cells and 22.6 g of docosahexaenoic acid (DHA content in total lipids, 39.2%).

(Inventive Example 6)

A 182.0 g portion of dry algal cells and 21.7 g of docosahexaenoic acid (DHA content in total lipids, 39.5%) were obtained by repeating the culturing of Inventive Example 4, except that a medium was prepared by adding 20 g/l of glucose as a carbon source and 2 g/l of yeast extract as nitrogen and vitamin sources to 7 L of a commercially available artificial sea water Aquamarine which was used as a mineral source, and the medium was supplied in such a way that the total carbon source used in the medium reached 10%.

(Inventive Example 7)

A 180.6 g portion of dry algal cells and 21.0 g of docosahexaenoic acid (DHA content in total lipids, 38.9%) were obtained by repeating the culturing of Inventive Example 4, except that a medium was prepared by adding 23 g/l of glucose as a carbon source and 2.0 g/l of yeast extract as nitrogen and vitamin sources to 7 L of sea water described in the following to be used as a mineral source, and the medium was supplied in such a way that the total carbon source used in the medium reached 10%.

(Comparative Example 1)

Using each of the marine microalgae shown in the following, culturing was carried out in the same manner as described in Inventive Example 1 to obtain docosahexaenoic acid. The results thus obtained are shown in Table 1 in terms of the amounts of algal cells and docosahexaenoic acid (DHA). For the sake of comparison, the results of Inventive Example 1 are also shown in Table 1.

8

Table 1

| Results of shaking culture (conical flasks) | | | |
|---|---|---|---|
| | C. cohnii ATCC | Amount of dry algal cells (g/l) | Amount of DHA (g/l) |
| Comparative Example 1 | 30543 | 1.7 | 0.21 |
| | 30556 | 1.6 | 0.19 |
| | 30571 | 1.7 | 0.22 |
| | 30572 | 1.6 | 0.20 |
| | 30775 | 1.4 | 0.16 |
| | 50051 | 1.7 | 0.22 |
| | 50053 | 1.7 | 0.19 |
| | 50055 | 1.6 | 0.17 |
| | 50056 | 1.5 | 0.15 |
| | 50058 | 1.6 | 0.20 |
| | 50060 | 1.7 | 0.20 |
| Inventive Example 1 | 30021 | 1.8 | 0.24 |

(Comparative Example 2)

Standing culturing was carried out under the same conditions as described in Inventive Example 1, except that the shaking culture was changed to standing culture. The results are shown in Table 2.

Table 2

| Results of standing culture | | |
|---|---|---|
| C. cohnii ATCC | Amount of dry algal cells (g/l) | Amount of DHA (g/l) |
| 30021 | 1.2 | 0.05 |
| 30543 | 1.1 | 0.04 |
| 30556 | 0.9 | 0.03 |
| 30571 | 1.0 | 0.04 |
| 30572 | 1.1 | 0.05 |
| 30775 | 1.1 | 0.04 |
| 50051 | 1.2 | 0.05 |
| 50053 | 1.0 | 0.03 |
| 50055 | 1.1 | 0.03 |
| 50056 | 0.9 | 0.05 |
| 50058 | 1.1 | 0.04 |
| 50060 | 1.2 | 0.05 |

(Inventive Example 8)

Culturing was carried out under the same conditions as described in Inventive Example 4 except that the antifoaming agent was not added to the culture medium. Though satisfactory culturing was not able to be carried out because of foaming, 4.6 g/l of dry algal cells and 0.39 g/l of docosahexaenoic acid (DNA content in total lipids, 33.2%) were obtained.

(Inventive Example 9)

Culturing was carried out under the same conditions as described in Inventive Example 4 except that the inoculum size for the culturing was changed to 1%.

Propagation of algal cells was weak, and 0.9 g/l of dry algal cells and 0.05 g/l of docosahexaenoic acid (DHA content in total lipids, 30.3%) were obtained.

(Inventive Example 10)

Culturing was carried out under the same conditions as described in Inventive Example 3 except that the inoculum size for the culturing was changed to 25%.

Dry algal cells were obtained with a yield of 42.1 g/l, and docosahexaenoic acid with a yield of 5.2 g (0.74 g/l) (DHA content in total lipids, 31.1%). These results were slightly inferior to the case of the use of 7% inoculum size (Inventive Example 3), and, when the inoculum size was changed to 25%, increase in the formation of docosahexaenoic acid was not observed and the amount of docosahexaenoic acid decreased relatively, thus entailing unstable production.

(Inventive Example 11)

Culturing was carried out under the same conditions as described in Inventive Example 3 except that the amount of the antifoaming agent (surfactant) was changed to 0.05 g.

Though foaming was observed with the progress of the culturing, 28.2 g of dry algal cells and 2.0 g of docosahexaenoic acid (0.29 g/l; DHA content in total lipids, 26.3%) were obtained.

(Inventive Example 12)

Culturing was carried out under the same conditions as described in Inventive Example 3 except that the amount of the antifoaming agent (surfactant) was changed to 7.5 g.

Propagation of algal cells was slightly weak though foaming was not observed, and 31.2 g of dry algal cells and 2.3 g of docosahexaenoic acid (0.33 g/l; DHA content in total lipids, 29.8%) were obtained.

(Inventive Examples 13 to 21)

A 100 ml portion of medium (II) shown in Table 5 was put in each of 300 ml capacity conical flasks, and one of the surfactants shown in Table 6 was added to the medium to a concentration of 0.05 g/L, followed by sterilization. After cooling, 5 ml of a culture broth of *Crypthecodinium cohnii* ATCC 30021, which has been obtained in advance by for 7 days of culturing in a medium prepared by dissolving 10 g/L of glucose and 2 g/L of yeast extract in an artificial sea water Aquamarine (manufactured by Yaesu Yakuhin) and adjusting its pH to 7.4, was inoculated to carry out 5 days of rotary shaking culture (180 rpm) at 28°C. Dry cell yields and docosahexaenoic acid contents obtained from the cultured algal cells are shown in Table 6.

(Inventive Examples 22 and 23)

Culturing was carried out in the same manner as described in Inventive Examples 13 to 21, except that surfactant was not used or the surfactant shown in Table 6 was used, with the results shown in Table 6.

(Inventive Examples 24 to 26)

Culturing was carried out in the same manner as described in Inventive Examples 13 to 21, except that Span 80 was added as a surfactant to the medium to a concentration shown in Table 7, with the results also shown in Table 7.

For the sake of convenience for comparison, results of Inventive Example 22 (no addition of surfactant) and Inventive Example 16 are also shown in the table.

Table 3   Composition of medium (I)

| NaCl | 23.48 g | FeCl$_3$·6H$_2$O | 0.01 g |
|---|---|---|---|
| MgCl$_2$·6H$_2$O | 10.63 g | Na$_2$ glycerophosphate | 0.15 g |
| Na$_2$SO$_4$ | 3.92 g | (NH$_4$)$_2$SO$_4$ | 0.05 g |
| CaCl$_2$ (anhydride) | 1.11 g | Tris buffer | 3.0 g |
| KCl | 0.66 g | vitamin mixture** | 1.0 ml |
| NaHCO$_3$ | 0.19 g | K$_2$HPO$_4$ | 0.01 g |
| KBr | 0.1 g | glucose | 20.0 g |
| H$_3$BO$_3$ | 0.03 g | glutamic acid | 1.5 g |
| SrCl$_3$·6H$_2$O | 0.04 g | casein hydrolysate | 20.0 g |
| trace mineral mixture* | 3.0 ml | distilled water | 1.0 L |

Note) *: trace mineral mixture

| Na$_2$EDTA | 1.0 g |
|---|---|
| FeCl$_3$·6H$_2$O | 0.05 g |
| H$_3$BO$_3$ | 1.0 g |
| MnCl$_2$·4H$_2$O | 0.15 g |
| ZnCl$_2$ | 0.01 g |
| CoCl$_2$·6H$_2$O | 0.005 g |
| distilled water | 100.0 ml |

**: vitamin mixture

| biotin | 0.003 g |
|---|---|
| thiamin | 1.0 g |
| distilled water | 1.0 L |

Table 4

| Composition of Aquamarine (Yaesu Yakuhin) | | | |
|---|---|---|---|
| MgSO$_4$·6H$_2$O | 11.11 g | KBr | 0.10 g |
| CaCl$_2$·2H$_2$O | 1.54 g | H$_3$BO$_3$ | 0.03 g |
| SrCl$_3$·6H$_2$O | 0.04 g | NaF | 0.003 g |
| KCl | 0.69 g | NaCl | 24.53 g |
| NaHCO$_3$ | 0.20 g | Na$_2$SO$_4$ | 4.09 g |
| distilled water | 1.0 L | | |
| pH 7.0 (adjusted with 1 N HCl) | | | |

(Sea water)

Sea water near the shore of Kawasaki-cho, Chuoh-ku, Chiba-shi, Japan, was collected and used.

Table 5

| Composition of medium (II) | |
|---|---|
| glucose | 20 g/L |
| corn steep liquor | 2 g/L |
| sodium glutamate | 2 g/L |
| NaCl | 20 g/L |
| $MgSO_4 \cdot 7H_2O$ | 10 g/L |
| $CaCl_2 \cdot 2H_2O$ | 1 g/L |
| $KNO_3$ | 1 g/L |
| $K_2HPO_4$ | 10 mg/L |
| $Na_2EDTA$ | 30 mg/L |
| $H_3BO_3$ | 30 mg/L |
| $FeCl_3$ | 1.5 mg/L |
| $MnCl_2 \cdot 4H_2O$ | 4.5 mg/L |
| $ZnSO_4$ | 0.63 mg/L |
| $CoCl_2 \cdot 6H_2O$ | 0.015 mg/L |
| Tris | 6 g/L |
| distilled water | 1 L |
| pH | 7.0 |

Table 6

| | Surfactant | HLB | Dry algal cell yield [g/L] | DHA yield [g/L] |
|---|---|---|---|---|
| Inventive Example 13 | Tween 80 | 15.00 | 4.7143 | 0.4277 |
| Inventive Example 14 | Tween 85 | 11.00 | 4.9938 | 0.4406 |
| Inventive Example 15 | Span 20 | 8.60 | 6.5361 | 0.4228 |
| Inventive Example 16 | Span 80 | 4.30 | 4.9212 | 0.5328 |
| Inventive Example 17 | Span 85 | 1.80 | 5.1480 | 0.4232 |
| Inventive Example 18 | oleic acid | 1.00 | 5.0386 | 0.4389 |
| Inventive Example 19 | oleyl alcohol oleic acid | 0.35 | 5.7128 | 0.4814 |
| Inventive Example 20 | monoglyceride oleic acid | 3.40 | 4.5381 | 0.5271 |
| Inventive Example 21 | triglyceride | -11.45 | 7.2344 | 0.4025 |
| Inventive Example 22 | no addition | - | 4.6344 | 0.3872 |
| Inventive Example 23 | Tween 20 | 16.90 | 4.6284 | 0.3147 |

Table 7

| | Concentration [g/L] | dry algal cell yield [g/L] | DHA yield [g/L] |
|---|---|---|---|
| Inventive Example 21 | - | 4.6344 | 0.3872 |
| Inventive Example 16 | 0.05 | 4.9212 | 0.5328 |
| Inventive Example 23 | 0.10 | 4.8015 | 0.5144 |
| Inventive Example 24 | 0.50 | 5.0034 | 0.5285 |
| Inventive Example 25 | 1.00 | 5.4758 | 0.5325 |

12

INDUSTRIAL APPLICABILITY

As has been described in the foregoing, since the prior art docosahexaenoic acid is obtained from fish oils such as of tuna, sardine and the like, its productivity, quality and the like vary depending on the fishing seasons and areas. However, docosahexaenoic acid having no fishy smells can be provided stably at a low cost by the process of the present invention in which *Crypthecodinium cohnii*, especially *Crypthecodinium cohnii* ATCC 30021, is cultured by liquid shaking culture, liquid submerged aeration agitation culture and/or aeration agitation culture using a medium supplemented with a surfactant and/or an antifoaming agent, especially a fatty acid ester surfactant having an HLB value of 16 or less. In addition to this, the docosahexaenoic acid obtained in this way does not contain other highly unsaturated fatty acids and therefore can be separated and extracted easily.

According to the culturing method of the present invention, especially when a medium supplemented with a surfactant is used, algal cells can be propagate stably, lipids in the algal cells hardly contain other highly unsaturated fatty acids and the content of docosahexaenoic acid can be increased.

**Claims**

1. A process for producing docosahexaenoic acid which comprises producing docosahexaenoic acid by culturing a marine microalga belonging to *Crypthecodinium cohnii* by means of liquid shaking culture, liquid submerged aeration agitation culture and/or aeration agitation culture in the presence of a surfactant and/or an antifoaming agent.

2. The process for producing docosahexaenoic acid according to claim 1 wherein said surfactant and/or antifoaming agent is a silicon surfactant and/or a fatty acid ester surfactant.

3. The process for producing docosahexaenoic acid according to claim 1 or 2 wherein said surfactant and/or antifoaming agent is used in an amount of from 0.01 to 1 g/l medium.

4. The process for producing docosahexaenoic acid according to any one of the claims 1 to 3 wherein said surfactant is a fatty acid ester surfactant having an HLB value of 16 or less.

5. The process for producing docosahexaenoic acid according to any one of the claims 1 to 4 wherein said culture is liquid submerged aeration agitation culture, and agitation blade peripheral speed is 250 cm/sec or less.

6. The process for producing docosahexaenoic acid according to any one of the claims 1 to 5 wherein said *Crypthecodinium cohnii* is *Crypthecodinium cohnii* ATCC 30021.

7. A process for producing docosahexaenoic acid which comprises culturing *Crypthecodinium cohnii* ATCC 30021 by means of liquid shaking culture, liquid submerged aeration agitation culture and/or aeration agitation culture.

8. A process for producing docosahexaenoic acid which comprises using a seed culture in an inoculum size of from 1.5 to 20% by weight based on an initial medium in any one of the docosahexaenoic acid production processes of claims 1 to 7.

9. *Crypthecodinium cohnii* algal cells containing docosahexaenoic acid, which are obtained by any one of the docosahexaenoic acid production processes of claims 1 to 8.

International application No.

PCT/JP93/00402

**A. CLASSIFICATION OF SUBJECT MATTER**

Int. Cl$^5$   C12P7/64

According to International Patent Classification (IPC) or to both national classification and IPC

**B. FIELDS SEARCHED**

Minimum documentation searched (classification system followed by classification symbols)

Int. Cl$^5$   C12P7/64

Documentation searched other than minimum documentation to the extent that such documents are included in the fields searched

Electronic data base consulted during the international search (name of data base and, where practicable, search terms used)

WPI, WPI/L

**C. DOCUMENTS CONSIDERED TO BE RELEVANT**

| Category* | Citation of document, with indication, where appropriate, of the relevant passages | Relevant to claim No. |
|---|---|---|
| X | Biochimica et Biophysica Acta; 712[3] p. 523-534 (1982) | 1-9 |
| X | Biochemica et Biophysica Acta; 316[1] p. 56-65 (1973) | 1-9 |
| A | JP, A, 2-23878 (Suntory Ltd.), February 28, 1990 (28. 02. 90), & EP, A, 355972 | 1-9 |

☐ Further documents are listed in the continuation of Box C.   ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| June 21, 1993 (21. 06. 93) | July 13, 1993 (13. 07. 93) |

| Name and mailing address of the ISA/ | Authorized officer |
|---|---|
| Japanese Patent Office | |
| Facsimile No. | Telephone No. |

Form PCT/ISA/210 (second sheet) (July 1992)